(19) Europäisches Patentamt
European Patent Office
Office européen des brevets

(11) Veröffentlichungsnummer: **0 271 013 B1**

# (12) EUROPÄISCHE PATENTSCHRIFT

(45) Veröffentlichungstag der Patentschrift: **09.06.93**

(51) Int. Cl.5: **C07C 255/37**, C07C 255/33, C07D 317/60, C07D 319/08, C07D 319/18, A61K 31/275, A61K 31/335

(21) Anmeldenummer: **87117946.1**

(22) Anmeldetag: **04.12.87**

## (54) Basisch substituierte Phenylacetonitrile, ihre Herstellung und diese enthaltende Arzneimittel.

(30) Priorität: **11.12.86 DE 3642331**

(43) Veröffentlichungstag der Anmeldung:
**15.06.88 Patentblatt 88/24**

(45) Bekanntmachung des Hinweises auf die Patenterteilung:
**09.06.93 Patentblatt 93/23**

(84) Benannte Vertragsstaaten:
**AT BE CH ES FR GB IT LI NL SE**

(56) Entgegenhaltungen:
**EP-A- 0 064 158**
**EP-A- 0 147 707**
**DE-A- 2 631 222**
**FR-A- 1 449 564**
**US-A- 4 593 042**

(73) Patentinhaber: **BASF Aktiengesellschaft**
**Carl-Bosch-Strasse 38**
**W-6700 Ludwigshafen(DE)**

(72) Erfinder: **Seitz, Werner, Dr.**
**Bismarckstrasse 22b**
**W-6831 Plankstadt(DE)**
Erfinder: **Unger, Liliane, Dr.**
**Waltraudenstrasse 14**
**W-6700 Ludwigshafen(DE)**
Erfinder: **Raschack, Manfred, Dr.**
**Donnersbergstrasse 7**
**W-6714 Weisenheim am Sand(DE)**
Erfinder: **Baldinger, Verena, Dr.**
**Schiffsgasse 6**
**W-6900 Heidelberg(DE)**
Erfinder: **Ruebsamen, Klaus, Dr.**
**Erschigweg 19**
**W-6730 Neustadt(DE)**
Erfinder: **Gries, Josef, Dr.**
**Roemerweg 43**
**W-6706 Wachenheim(DE)**

EP 0 271 013 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue basisch substituierte Phenylacetonitrile, deren Herstellung sowie Arzneimittel, welche diese Substanzen enthalten.

In DE-PS 1 544 810, DE-PS 1 493 904, DE-OS 15 93 921, DE-OS 16 43 429, EP-OS 147 707, EP-OS 64 158, EP-OS 157 206, DE-OS 35 38 063, DE-OS 35 37 715, EP-OS 180 810, JP-OS 146 001 = Derwent No. 86-078808/12, JP-OS 000 304 = Derwent No. 85-226650/37 und US-PS 4 593 042 sind basisch substituierte Phenylacetonitrile beschrieben. Aus dieser Verbindungsklasse haben sich aufgrund ihrer calciumantagonistischen Wirkung Verapamil (X = H) und Gallopamil (X = $OCH_3$),

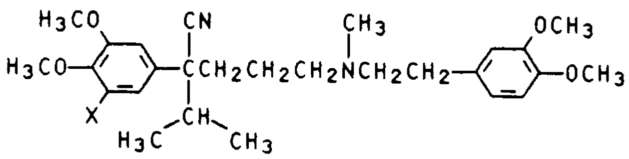

in der Therapie der coronaren Herzkrankheiten und des Bluthochdrucks bewährt.

Für das Verapamilmolekül sind Zusammenhänge zwischen chemischer Struktur und biologischer Wirkung verschiedentlich publiziert worden (Arzneim. Forsch./Drug Res. 5 (1981), 773).

Aufgrund dieser Struktur-Wirkungs-Betrachtungen und experimenteller Arbeiten wurde von Mannhold [Drugs of Today 20(2), 69-90 (1984)] nachgewiesen, daß beide Arylreste des Verapamilmoleküls essentiell für die biologische Wirkung sind.

Die in den vorstehenden Patentschriften aufgeführten Verbindungen zeichnen sich alle dadurch aus, daß der basische Stickstoff durch eine niedermolekulare Alkylgruppe und auf der rechten Seite des Stickstoffs durch eine Aralkylgruppe substituiert ist. Der basische Stickstoff und der Arylrest sind durch mindestens zwei Kohlenstoffatome getrennt, die auch Teil eines partiell hydrierten bicyclischen aromatischen oder heteroaromatischen Substituenten (z.B. Tetrahydronaphthyl- bzw. Isochromanring) oder Bestandteil eines Ringes (z.B. Piperidinring) sein können.

Auch weitergehende Abwandlungen des Verapamils, wie sie z.B. in den DE-OS 25 09 797 und DE-OS 24 60 593 beschrieben sind, enthalten als essentielles Strukturelement die Phenethylaminseitenkette.

Es wurde gefunden, daß neue basisch substituierte Phenylacetonitrile der Formel I

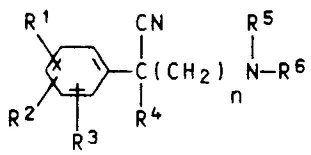

I,

worin

- $R^1$, $R^2$, $R^3$ gleich oder verschieden sind und Wasserstoffatome, Halogenatome, Trifluormethylgruppen, $C_1$-$C_4$-Alkylgruppen, Nitrogruppen oder $C_1$-$C_4$-Alkoxygruppen bedeuten, wobei auch jeweils zwei Reste in Nachbarstellung zusammen Methylendioxy-, Ethylendioxy-, 1,3-Dioxatetramethylen-,Propylen- oder Butylengruppen bilden können,
- $R^4$ eine gesättigte oder ungesättigte Alkylgruppe, eine Cycloalkyl- oder Phenylgruppe ist,
- $R^5$ ein Wasserstoffatom oder einen $C_1$-$C_4$-Alkylrest darstellt,
- $R^6$ einen gesättigten oder ungesättigten Kohlenwasserstoffrest mit 6 bis 15 Kohlenstoffatomen, der gegebenenfalls einen nicht-aromatischen Ring enthalten kann, darstellt und
- n die Zahl 2, 3 oder 4 bedeutet,

sowie deren Antipoden und Salze mit physiologisch verträglichen Säuren trotz Fehlen des Arylrestes in der Phenalkylseitenkette hochwirksam sind.

Als Halogenatome für $R^1$, $R^2$ und $R^3$ kommen bevorzugt Fluor- und Chloratome in Betracht. Bevorzugte Alkyl- und Alkoxygruppen für $R^1$ bis $R^3$ sind solche mit 1 bis 2 Kohlenstoffatomen. Bevorzugte Nitro- und Trifluormethylverbindungen sind solche mit einer Nitro- bzw. Trifluormethylgruppe. Der Rest $R^4$ enthält vorzugsweise bis zu 6 Kohlenstoffatome.

Besonders interessant sind die folgenden Verbindungen:

5-[N-Methyl-N-(n-octyl)]amino-2-(3,4,5-trimethoxyphenyl)-2-isopropyl-valeronitril und seine Enantiomeren,

5-[N-Methyl-N-(n-hexyl)]amino-2-(3,4,5-trimethoxyphenyl)-2-isopropyl-valeronitril und seine Enantiomeren,
5-[N-Methyl-N-(2-cyclohexylethyl)]amino-2-(3,4,5-trimethoxyphenyl)-2-isopropyl-valeronitril und seine Enantiomeren,
5-[N-Methyl-N-(2-cyclohexylethyl)]amino-2-phenyl-2-isopropyl-valeronitril und seine Enantiomeren,
5-{N-Methyl-N-[2-(cyclohexen-1-yl)ethyl]}amino-2-(3,4,5-trimethoxyphenyl)-2-isopropyl-valeronitril und seine Enantiomeren,
5-{N-Methyl-N-[2-(cyclohexen-1-yl)ethyl]}amino-2-phenyl-2-isopropyl-valeronitril und seine Enantiomeren.

Als physiologisch verträgliche Säuren kommen z.B. in Frage: Salzsäure, Bromwasserstoffsäure, Schwefelsäure, Phosphorsäure, Essigsäure, Maleinsäure, Milchsäure, Weinsäure, Zitronensäure und Fumarsäure.

Die neuen Verbindungen besitzen mindestens ein asymmetrisches C-Atom und liegen daher in den verschiedenen enantiomeren Formen vor. Folglich können die Verbindungen I entweder in optisch aktiven Formen oder als racemische Mischungen hergestellt werden. Die Racemate der Verbindungen I können durch herkömmliche Techniken, z.B. durch Trennung (fraktionierte Kristallisation, Säulenchromatographie) der diastereomeren Salze, in ihre optische Antipoden gespalten werden. Die diastereomeren Salze sind durch Umsetzung der Verbindungen I mit chiralen Säuren herstellbar. Die enantiomeren Formen können auch durch die Verwendung optisch aktiver Ausgangsverbindungen erhalten werden.

Die neuen Verbindungen werden hergestellt, indem man

a) Phenylacetonitrile der Formel II

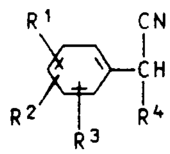

II,

worin $R^1$ bis $R^4$ die angegebene Bedeutung haben, mit Aminen der Formel III

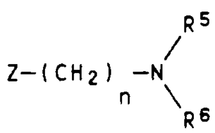

III,

worin $R^5$, $R^6$ und n die angegebene Bedeutung haben und Z eine Austrittsgruppe darstellt, umsetzt oder

b) basisch substituierte Phenylacetonitrile der Formel IV

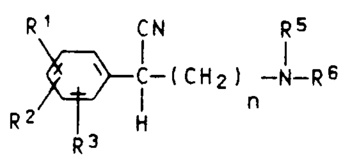

IV,

worin $R^1$ bis $R^3$, $R^5$, $R^6$ und n die angegebene Bedeutung haben mit Verbindungen der Formel V

$R^4$-Z V,

worin $R^4$ und Z die vorstehende Bedeutung haben, zur Reaktion bringt oder

c) Phenylacetonitrile der Formel VI

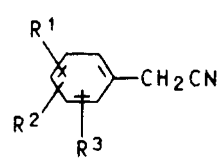

VI,

worin $R^1$ bis $R^3$ die angegebene Bedeutung haben, mit Aminen der Formel III und Verbindungen der Formel V umsetzt oder

d) Phenylacetonitrile der Formel VII

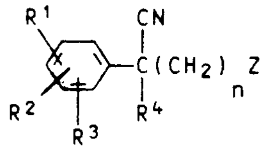

VII,

worin $R^1$ bis $R^4$, n und Z die oben angegebene Bedeutung haben, mit einem Alkylamin der Formel VIII

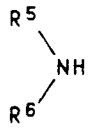

VIII,

worin $R^5$ und $R^6$ die obige Bedeutung haben, zur Reaktion bringt oder

(e) Phenylacetonitrile der Formel IX

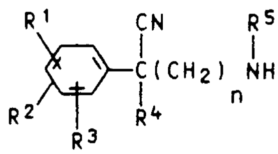

IX,

bzw. der Formel X

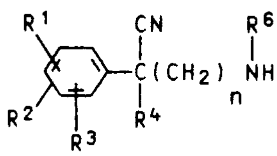

X,

worin $R^1$ bis $R^6$ und n die beschriebene Bedeutung haben, mit Verbindungen der Formel XI bzw. XII

XI $R^6$-Z bzw. XII $R^5$-Z

worin $R^5$, und $R^6$ und Z die oben angegebene Bedeutung (ausgenommen $R^5$ = H) haben, zur Reaktion bringt oder

f) - falls die Reste $R^5$ und $R^6$ eine mit dem Stickstoffatom verbundene $CH_2$-Gruppe enthalten - Phenylacetonitrile der Formel IX bzw. X mit Aldehyden der Formel XIII bzw. XIV

XIII $R^7$-CHO bzw. XIV $R^8$-CHO

wobei $R^7$ bzw. $R^8$ so definiert sind, daß $R^7$-$CH_2$ gleich $R^5$ und $R^8$-$CH_2$ gleich $R^6$ bedeuten, unter reduktiven Bedingungen umsetzt oder

g) Aldehyde der Formel XV

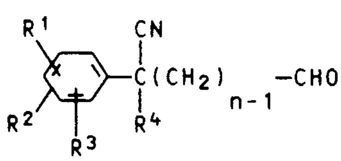

XV,

wobei $R^1$ bis $R^4$ und n dasselbe wie oben bedeuten mit Alkylaminen der Formel VIII unter reduktiven Bedingungen umsetzt oder

h) Dinitrile der Formel XVI

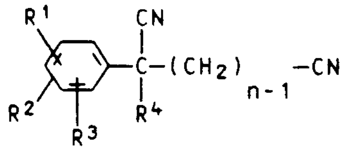

XVI,

worin $R^1$ bis $R^4$ und n dasselbe wie oben bedeuten in Gegenwart eines Alkylamins der Formel VIII reduziert oder

i) Nitrile der Formel XVII bzw. XVIII

$R^7$-CN XVII bzw. $R^8$-CN XVIII

wobei $R^7$ bzw. $R^8$ wie vorstehend definiert sind und $R^7$ nicht Wasserstoff bedeuten kann, mit Phenylacetonitrilen der Formel IX bzw. X hydriert oder

k) - falls $R^6$ eine mit dem Stickstoffatom verbundene $CH_2$-Gruppe enthält - Phenylacetonitrile der Formel XIX

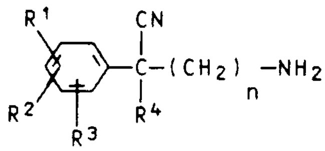

XIX,

worin $R^1$ bis $R^4$ und n dasselbe wie oben bedeuten, in Gegenwart von Nitrilen der Formel XVIII hydriert und in den so erhaltenen Verbindungen, falls $R^5$ ein Wasserstoffatom darstellt, gewünschtenfalls eine $C_1$-$C_4$-Alkylgruppe einführt und anschließend die Verbindungen gegebenenfalls in ihre Salze mit physiologisch verträglichen Säuren überführt.

Die Umsetzung a) kann beispielsweise durchgeführt werden, indem man ein CH-acides Phenylacetonitril der Formel II in einem inerten Lösungsmittel mit einer Base metalliert und anschließend mit Verbindungen der Formel III umsetzt. Gegebenenfalls kann auch so verfahren werden, daß die Base zu einer Lösung von Verbindungen der Formel II und III zugegeben wird.

Als Basen kommen in Betracht: Alkalimetall-hydride, -hydroxide, -alkoholate, -amide und metallorganische Verbindungen. Vorzugsweise werden verwendet: Natriumamidpulver und -suspension, Kaliumhydroxidpulver, Butyllithium, Lithiumdiisopropylamid.

Als Lösungsmittel für die Reaktion eignen sich aromatische und aliphatische Kohlenwasserstoffe, jedoch sind auch höhersiedende aliphatische Ether und dipolar aprotische Lösungsmittel geeignet. Bevorzugt wird mit Toluol gearbeitet.

Reaktion a) kann auch nach einem phasentransferkatalysierten Verfahren durchgeführt werden. Als Katalysatoren finden quartäre Ammonium-, Phosphoniumsalze, Kronenether, Polyethylenglykol-dialkylether (z.B. PEG 600-dibutylether) und Tris-(3,6-dioxaheptyl)amin (TDA-1) Verwendung.

Die Reaktionstemperaturen sind von den eingesetzten Basen abhängig, z.B. wird mit Butyllithium bei Temperaturen zwischen 0 und -100 °C und bei Verwendung von Natriumamid vorzugsweise bei 50 bis 150 °C gearbeitet.

Als Austrittsgruppen Z eignen sich beispielsweise Chlor, Brom, Schwefelsäureester, Tosylate, Mesylate oder Triflate.

Die Umsetzung von Verbindungen der Formel IV zu den erfindungsgemäßen Verbindungen (Verfahren b) erfolgt in ähnlicher Weise wie bei Verfahren a).

Als Reaktionspartner kommen Alkanderivate der Formel V in Frage, wobei die Austrittsgruppe Z die gleiche Bedeutung wie oben hat.

Bei Verfahren c) kann die Reihenfolge der Zugabe der Verbindungen III, V und VI beliebig gewählt und auf eine Isolierung von Zwischenprodukten verzichtet werden.

Die Reaktion d) erfolgt durch einfaches Erhitzen der Reaktionspartner auf vorzugsweise 120 bis 180 °C. Sie kann auch in einem Lösungsmittel erfolgen, doch ist dieses nicht erforderlich. Dasselbe gilt für die Umsetzung e). Als Z eignet sich in beiden Fällen Halogen, vorzugsweise Chlor und Brom, jedoch sind auch Mesylate und Tosylate geeignet.

Die Umsetzungen d) und e) werden bevorzugt in einem dipolar aprotischen Lösungsmittel, wie z.B. Acetonitril, Dimethylformamid, Hexamethylphosphorsäuretriamid, unter Zusatz eines säurebindenden Mittels, z.B. wasserfreies Kaliumcarbonat oder Triethylamin, durchgeführt. Als säurebindendes Mittel kann auch ein zusätzliches Äquivalent des Amins (Formel VIII, IX oder X) eingesetzt werden. Die Reaktionstemperatur kann von Raumtemperatur bis 120 °C gewählt werden. Bevorzugt wird die Reaktion zwischen 70 und 100 °C durchgeführt.

Beim Verfahren f) und g) werden die Aldehyde der Formel XIII, XIV und XV mit den Aminen VIII, IX und X in einer Kondensation unter reduktiven Bedingungen umgesetzt.

Als Lösungsmittel sind aliphatische und aromatische Kohlenwasserstoffe, halogenierte Kohlenwasserstoffe, Ether, Alkohole oder niedrige Fettsäuren geeignet. Die Reaktionstemperaturen liegen zwischen 0 und 150 °C, vorzugsweiswe 20 bis 70 °C.

Als Reduktionsmittel kommen in Betracht: Wasserstoff in Gegenwart eines Katalysators, z.B. $PtO_2$, Pd/C, Nickel- oder Kobalt-Katalysatoren, nascierender Wasserstoff, den man aus Metall und Säure erhält, komplexe Metallhydride (z.B. $NaBH_4$) oder Hydriddonatoren (z.B. Ameisensäure).

Wird die Reduktion in Gegenwart eines Katalysators durchgeführt, so arbeitet man bevorzugt bei Atmosphärendruck.

Die Methylierung von Verbindungen der Formel X kann auch nach Leukart-Wallach mit Formaldehyd/Ameisensäure durchgeführt werden.

Die Reduktion des Dinitrils der Formel XVI in Gegenwart eines Alkylamins der Formel VIII, worin $R^5$ auch Wasserstoff sein kann (Umsetzung h und m) bzw. von Nitrilen der Formel XVII und XVIII in Gegenwart von Phenylacetonitrilen der Formel IX und X (Umsetzung i und k) erfolgt vorzugsweise als katalytische Hydrierung mit einem Edelmetallkatalysator, vorzugsweise Pd/C. Die Reaktionstemperaturen liegen zwischen 30 und 80 °C, vorzugsweise bei 60 °C. Die Reaktion kann unter Normaldruck oder Überdruck bis 6 bar erfolgen. Als Lösungsmittel werden niedrige Alkohole, Essigsäure oder aromatische Kohlenwasserstoffe, vorzugsweise niedrige aliphatische Alkohole, wie Ethanol, verwendet. Bezogen auf das eingesetzte Amin sind 0,1 bis 10 Gew.% Pd/C-Katalysator erforderlich, wobei der Katalysator 1 bis 10 Gew.% Pd auf Kohlenstoff enthält.

Die vorstehenden Reaktionen und die Herstellung der Ausgangsstoffe sind beschrieben in: DE-PS 1 154 810, DE-PS 1 493 904, DE-PS 1 158 083, DE-PS 2 059 923, DE-OS 22 63 527, DE-PS 2 631 222, DE-OS 30 34 221, EP 165 322, EP 64 148 und EP 47 888.

Die Verbindungen der allgemeinen Formel I bzw. deren physiologische Säureadditionssalze haben wertvolle pharmakologische Eigenschaften. Sie wirken als hochaktive Ca-Antagonisten dilatatorisch auf periphere und zentrale Gefäße und schützen Gehirn, periphere Organe (wie Herz und Niere) und Gefäße vor Schäden, die durch gesteigerte Ca-Mobilisierung bzw. Ca-Überladung wie z.B. bei Hypoxie und Ischämie hervorgerufen werden. Darüber hinaus hemmen die Verbindungen die Magensäuresekretion und besitzen cytoprotektive und antiulceröse Wirkung. Schließlich sind sie in der Lage, Spasmen der Bronchialmuskulator zu verhindern oder zu lösen.

Zusätzlich zeigen diese Verbindungen eine starke Antiserotonin-Wirkung. Es ist bekannt, daß Serotonin an der Entstehung von Spasmen zentraler und peripherer Gefäße sowie bei der zu Gefäßverschlüssen führenden Blutplättchenaggregation beteiligt ist und dabei je nach klinischem Bild die Bedeutung der durch Ca-Ionen bedingten Erregungen sogar übertreffen kann. Bei solchen Erkrankungen finden sowohl Ca-Antagonisten als auch Serotonin-Antagonisten Verwendung. Es ist deshalb von therapeutischem Wert, beide Wirkprinzipien in einer molekularen Struktur vereint zu haben.

Die erfindungsgemäßen Verbindungen sind daher geeignet zur Behandlung von Herzkreislauferkrankungen, insbesondere coronarer Herzerkrankung, Vasospasmen, cerebraler Ischämie, Hypertonie und Kreislaufschock. Weiterhin können sie zur Prophylaxe und zur Therapie von Magen- und Duodenalulcera sowie asthmatischer Erkrankungen verwendet werden.

Die Verbindungen können in üblicher Weise oral oder parenteral gegeben werden. Die Dosierung hängt vom Alter, Zustand und Gewicht des Patienten sowie von der Applikationsart ab. In der Regel beträgt die tägliche Wirkstoffdosis zwischen etwa 0,1 und 10 mg/kg Körpergewicht bei parenteraler Gabe. Im Normalfall werden tägliche Dosen von 1 bis 5 mg/kg oral und 0,05 bis 0,25 mg/kg parenteral angewendet.

Die Wirkqualität der erfindungsgemäßen Substanzen wurde mit folgenden Tests erfaßt:

a) Bindung an den Kalzium-Kanal Verdrängung der spezifischen (S)-$^3$H-Devapamil-Bindung in Membranpräparationen von Meerschweinchen-Skelettmuskel

b) Bindung an den Serotonin-$S_2$-Rezeptor Verdrängung der spezifischen $^3$H-Ketanserin-Bindung in Membranpräparationen von Cerebral-Cortex der Ratte

Membranhomogenat wurde mit steigenden Konzentrationen ($10^{-10}$-$10^{-6}$ M) von Prüfsubstanz und einer festen Konzentration von 1 nM Radioligand ((S)-$^3$H-Devapamil bzw. $^3$H-Ketanserin) 60 Minuten bei Raumtemperatur inkubiert. Der gebundene und der freie Radioligand wurde durch Filtration über Glasfaserfilter getrennt und die Menge des auf dem Filter zurückgehaltenen Radioliganden mittels Flüssigkeitszintillationsmessung bestimmt. Es wurden 2 Versuche in 3fachem Ansatz durchgeführt.

Die Kompetitionskonstante ($K_i$-Werte in nM) wurde durch nicht-lineare Regressionsanalyse an einem IBM-Rechner in Anlehnung an das Programm Ligand von Munson und Rodbard (Analytical Biochemistry 107, 220, 1980) berechnet.

Wie die Tabelle zeigt, sind die Kompetitionskonstanten der erfindungsgemäßen Substanzen im Test auf Kalzium-Kanal-Bindung und Serotonin-$S_2$-Rezeptorbindung niedriger als die der Vergleichssubstanz Verapamil. Dementsprechend ist die Affinität der erfindungsgemäßen Substanzen zum Kalzium-Kanal bis zu 14fach und zum Serotonin-$S_2$-Rezeptor bis zu 63fach höher.

Tabelle

| Substanz des Beispiels Nr. | $K_i$ (nM) (S)-$^3$H-Devapamil-Verdrängung | $^3$H-Ketanserin-Verdrängung |
|---|---|---|
| 1 | 16,2 | 5 |
| 2 | 19,1 | 21 |
| 4 | 5,2 | 26,4 |
| 5 | 11,9 | 20 |
| 6 | 2,9 | 11,2 |
| 10 | 9,2 | 2,8 |
| 11 | 5,8 | 10,7 |
| 13 | 3,4 | 3,3 |
| 15 | 7,5 | 6,8 |
| 16 | 34,2 | 3,6 |
| 18 | 36,7 | 4 |
| 23 | 11,6 | 48,4 |
| 24 | 8,6 | 21,1 |
| Verapamil | 41 | 177 |

Die erfindungsgemäßen Wirkstoffe können in die üblichen galenischen Anwendungsformen, wie Tabletten, Dragees, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk und/oder Mitteln zur Erzielung eines Depoteffektes, wie Carboxipolymethylen, Carboximethylencellulose, Celluloseacetatphalat oder Polyvinylacetat erhalten werden.

Die Tabletten können auch aus mehreren Schichten bestehen. Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmacksverbesserndes Mittel, z.B. Aromastoffe, wie Vanillin oder Orangenextrakt, enthalten.

Sie können außerdem Suspendierhilfsstoffe oder Dickungsmittel, wie Natriumcarboximethylcellulose, Netzmittel, beispielsweise Kondensationsprodukte von Fettalkoholen mit Ethylenoxid oder Schutzstoffe, wie p-Hydroxibenzoate, enthalten.

Injektionslösungen werden in üblicher Weise, z.B. unter Zusatz von Konservierungsmitteln, wie p-Hydroxibenzoaten oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit mischt und in Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyethylenglykol bzw. dessen Derivaten, herstellen.

Die folgenden Beispiele erläutern die Erfindung, ohne sie zu beschränken:

## Beispiel 1

5-[N-Methyl-N-(2-cyclohexyl)ethyl]amino-2-(3-methoxyphenyl)-2-isopropyl-valeronitril

Eine Lösung von 18,9 g (0,1 Mol) α-Isopropyl-3-methoxyphenylacetonitril und 21,8 g (0,1 Mol) N-(3-Chlorpropyl)-N-methyl-2-cyclohexylethylamin in 100 ml Toluol wurde unter Rühren auf 85 °C erwärmt. Anschließend wurden innerhalb von 2 h 8 g (0,1 Mol) 50 %ige toluolische Natriumamidsuspension zugetropft. Die Reaktionslösung wurde 15 min bei 85 °C nachgerührt und nach dem Erkalten mit 200 ml Eiswasser versetzt. Die Toluolphase wurde abgetrennt, zweimal mit Wasser gewaschen und anschließend das Toluol im Vakuum abdestilliert. Der ölige Rückstand wurde in 150 ml Ethanol gelöst und mit isopropanolischer Salzsäure versetzt. Das ausgefallene Hydrochlorid wurde abgesaugt und aus 200 ml Isopropanol umkristallisiert. Man isolierte 34,6 g (85 %) Hydrochlorid, Fp. 144-145 °C.

## Beispiel 2

5-[N-Methyl-N-(n-decyl)]amino-2-(3,4,5-trimethoxyphenyl)-2-isopropyl-valeronitril

41,9 g (0,1 Mol) 5-[N-Methyl-N-(n-decyl)]amino-2-(3,4,5-trimethoxyphenyl)-valeronitril wurden in 200 ml Toluol gelöst und mit 4,7 g (0,12 Mol) pulverisiertem Natriumamid 1 h unter Rühren und Rückfluß erhitzt. Anschließend tropfte man innerhalb von 60 min eine Lösung von 15,7 g (0,12 Mol) Isopropylbromid in 30 ml Toluol zu und erhitzte noch 2 h unter Rückfluß. Die erkaltete Reaktionsmischung wurde in Wasser gegossen, die Toluolphase mehrmals mit Wasser gewaschen und anschließend das Toluol abdestilliert. Der Rückstand wurde in 100 ml Isopropanol gelöst und mit ethanolischer Salzsäure versetzt. Nach Absaugen wurde das Hydrochorid aus Ethanol umkristallisiert. Man isolierte 45,7 g (92 %) Hydrochlorid, Fp. 116-117 °C.

## Beispiel 3

5-[N-Methyl-N-(n-octyl)]amino-2-phenyl-2-isopropyl-valeronitril

11,7 g (0,1 Mol) Phenylacetonitril wurden in 15 ml Toluol gelöst und mit 52 g (0,8 Mol) 85 %igem Kaliumhydroxidpulver und 0,2 g Tris-(3,6-dioxaheptyl)amin versetzt. Anschließend wurden unter Rühren 12,3 g Isopropylbromid so zugetropft, daß die Reaktionstemperatur 50 °C nicht überstieg. Nach beendeter Zugabe wurde 30 min bei 50 °C nachgerührt und anschließend eine Lösung von 22,0 g (0,1 Mol) N-(3-Chlorpropyl)-N-methyl-octylamin in 200 ml Toluol bei 90 °C zugegeben. Die Reaktionsmischung wurde weitere 3 h bei 90 °C gerührt, nach Erkalten mit 100 ml Wasser versetzt und die Toluolphase abgetrennt. Nach Abdestillieren des Toluols erhielt man ein gelbes Öl, das über eine Kieselgelsäule mit einem Lösungsmittelgemisch aus Methylenchlorid/Aceton/Methanol (9/1/0,5) chromatographiert wurde. Man isolierte 24,0 g Base.

| Analyse: | C | H | N |
|---|---|---|---|
| ber. | C 80,6 | H 11,2 | N 8,2 |
| gef. | C 80,5 | H 11,1 | N 8,2 |

Beispiel 4

5-[N-Methyl-N-(n-octyl)]amino-2-(3,4,5-trimethoxyphenyl)-2-isopropyl-valeronitril

41,9 g (0,1 Mol) 5-[N-(n-Octyl]amino-2-(3,4,5-trimethoxyphenyl)-2-isopropyl-valeronitril wurden in der Kälte in 50 ml Ameisensäure gelöst und nach Zugabe von 11,9 ml 35 %iger wäßriger Formalinlösung (0,15 Mol) bis zur Beendigung der Kohlendioxidentwicklung auf dem Wasserbad erhitzt. Nach dem Abkühlen wurde die Reaktionslösung mit Wasser verdünnt, durch Zugabe von Ammoniak alkalisch gemacht und mit Ether die abgeschiedene Base extrahiert. Die etherische Lösung wurde mehrmals mit Wasser gewaschen und mit Kaliumcarbonat getrocknet. Anschließend wurde der Ether abdestilliert. Der Rückstand wurde über eine Kieselgelsäule chromatographiert und anschließend in das Hydrochlorid überführt. Man erhielt 43,2 g (92 %), Fp. 169-171 °C.

Beispiel 5

5-[N-Methyl-N-(2-cyclohexyl)ethyl]amino-2-(3,5-diethoxyphenyl)-2-isopropyl-valeronitril

32,4 g (0,1 Mol) α-Isopropyl-α-(3-chlorpropyl)-3,5-diethoxyphenylacetonitril und 14,1g (0,1 Mol) N-Methyl-2-cyclohexylethylamin wurden in 45 ml Hexamethylphosphorsäuretriamid gelöst und mit 30 g wasserfreiem gepulvertem Kaliumcarbonat versetzt. Das Reaktionsgemisch wurde 4 h auf 80 °C erhitzt, nach Abkühlen mit 500 ml Wasser versetzt und zweimal mit Ether extrahiert. Die Etherphase wurde mehrmals mit Wasser gewaschen, über Kaliumcarbonat getrocknet und der Ether abdestilliert. Die erhaltene Rohbase wurde chromatographisch gereinigt. Man isolierte 32,2 g (75 %) Base.

| Analyse: | C | H | N |
|---|---|---|---|
| ber. | C 75,6 | H 10,3 | N 6,5 |
| gef. | C 75,5 | H 10,1 | N 6,6 |

Beispiel 6

(S)-5-[N-Methyl-N-(n-octyl)]amino-2-(3,4,5-trimethoxyphenyl)-2-isopropyl-valeronitril

38,5 g (0,1 Mol) (S)-α-Isopropyl-α-(3-methansulfonyloxypropyl)-3,4,5-trimethoxyphenyl-acetonitril (Fp. 101 °C, $[\alpha]_D^{20}$ = 20 °, c = 10 mg/ml, EtOH, d = 10 cm), 31,5 g N-Methyl-octylamin und 0,5 g Tetrabutylammoniumbromid wurden in 100 ml Acetonitril 2,5 h auf 60 °C erwärmt. Nach Abkühlen wurde das Reaktionsgemisch in 200 ml Wasser gegossen und zweimal mit je 150 ml n-Hexan extrahiert. Die n-Hexanphase wurde mehrmals mit wäßriger Kochsalzlösung gewaschen und mit Kaliumcarbonat getrocknet und anschließend das n-Hexan abdestilliert. Die Rohbase wurde in einem Ether-Diisopropylether-Gemisch (3/2) gelöst und bis zum leicht sauren pH-Wert mit ethanolischer Salzsäure versetzt. Man isolierte 33,8 g (78 %) Hydrochlorid,
Fp. 131-133 °C,
(c = 10 mg/ml, Ethanol, d = 10 cm)

Beispiel 7

(R)-5-[N-Methyl-N-(n-octyl)]amino-2-(3,4,5-trimethoxyphenyl)-2-isopropyl-valeronitril

Analog Beispiel 5 wurden aus 32,6 g (0,1 Mol) (R)-α-Isopropyl-α-(3-chlorpropyl)-3,4,5-trimethoxyphenylacetonitril (DE-PS 2 059 985) und 14,3 g (0,1 Mol) N-Methyl-octylamin der rechtsdrehende

$$[\alpha]_{589}^{20} = +8,8^0$$

Antipode in Form des Hydrochlorids erhalten, Fp. 131-133 °C,
(c = 10 mg/ml, EtOH, d = 10 cm)

Beispiel 8

5-[N-(2-Cyclohexyl)ethyl]amino-2-(3,5-diethoxyphenyl)-2-isopropyl-valeronitril

Analog Beispiel 5 wurden aus 30,4 g (0,1 Mol) 5-Amino-2-(3,5-diethoxyphenyl)-2-isopropyl-valeronitril und 19,1 g (0,1 Mol) 2-Bromethyl-cyclohexan nach chromatographischer Reinigung 25,7 g (62 %) Base isoliert.

| | | | |
|---|---|---|---|
| Analyse: ber. | C 75,3 | H 10,2 | N 6,8 |
| gef. | C 75,4 | H 10,0 | N 6,7 |

Beispiel 9

5-[N-Methyl-N-(2-cyclohexyl)ethyl]amino-2-phenyl-2-isopropyl-valeronitril

21,5 g (0,1 Mol) 4-Phenyl-4-cyano-5-methylhexanal und 14,1 g (0,1 Mol) N-Methyl-2-cyclohexylethylamin wurden in 100 ml Toluol gelöst. Dazu gab man in der Kälte 4,6 g (0,1 Mol) Ameisensäure. Das Reaktionsgemisch wurde bis zum Abklingen der Gasentwicklung unter Rückfluß erhitzt. Die erkaltete Reaktionslösung wurde mit wäßriger Kaliumcarbonatlösung versetzt, das freigesetzte Amin mit Ether extrahiert und die etherische Phase mehrmals mit Wasser gewaschen. Nach Trocknen und Abdestillieren des Ethers wurde die ölige Base in Isopropanol gelöst und mit ethanolischer Salzsäure versetzt. Man erhielt nach Umkristallisieren aus Isopropanol 34,7 g Hydrochlorid, Fp. 142-144 °C.

Beispiel 10

5-[N-Methyl-N-(2-cyclohexyl)ethyl]amino-2-(3,5-dimethoxyphenyl)-2-isopropyl-valeronitril

Ein Gemisch aus 29,0 g (0,1 Mol) 2-(3,5-Dimethoxyphenyl)-2-isopropyl-5-methylamino-valeronitril (hergestellt durch katalytische Hydrierung von 2-(3,5-Dimethoxyphenyl)-2-isopropyl-1,3-dicyanopropan und anschließende N-Methylierung) und 12,6 g Cyclohexylacetaldehyd wurde mit 400 mg 5 %igem Palladium auf Kohle in 100 ml Toluol katalytisch unter Atmosphärendruck bei 25 bis 30 °C 10 h reduziert. Nach Entfernung des Katalysators wurde die toluolische Lösung mehrmals mit Wasser gewaschen und mit Kaliumcarbonat getrocknet. Anschließend wurde das Toluol abdestilliert. Der Rückstand wurde säulenchromatographisch gereinigt. Man isolierte 32,1 g (80 %) farblose Base.

| | | | |
|---|---|---|---|
| Analyse: ber. | C 75,0 | H 10,1 | N 7,0 |
| gef. | C 75,3 | H 10,1 | N 7,1 |

Beispiel 11

5-[N-Methyl-N-(2-cyclohexyl)ethyl]amino-2-(3,4,5-trimethoxyphenyl)-2-isopropyl-valeronitril

In einer Hydrierapparatur wurden 30,2 g 2-(3,4,5-Trimethoxyphenyl-2-isopropyl-1,3-dicyanopropan in 150 ml Isopropanol gelöst, mit 4 g 5 %igem Pd/C versetzt und mit Stickstoff gespült. Nach Zugabe von 2 ml wäßriger Cetyl-trimethyl-ammoniumchlorid-Lösung wurde unter kräftigem Rühren ein Wasserstoffstrom durch die Apparatur geleitet und das Vorratsgefäß mit Wasserstoff gefüllt. Dann gab man 14,1 g (0,1 Mol) N-Methyl-2-cyclohexylethylamin zu dem Reaktionsgemisch und hydrierte bei 50 °C bis 60 °C unter leichtem Wasserstoffüberdruck. Nach 2 h war die Hydrierung beendet. Nach Abtrennen des Katalysators wurde das Isopropanol abdestilliert, der Rückstand in Essigester aufgenommen und mit ethanolischer Salzsäure versetzt. Man isolierte 39,7 g (85 %) Hydrochlorid, Fp. 171-173 °C.

Beispiel 12

5-[N-(2-Cyclohexyl)ethyl]amino-2-(3,4,5-trimethoxyphenyl)-2-isopropyl-valeronitril

Analog Beispiel 11 wurden aus 30,6 g (0,1 Mol) 5-Amino-2-(3,4,5-trimethoxyphenyl)-2-isopropyl-valeronitril und 12,3 g (0,1 Mol) Cyclohexylacetonitril 35,4 g (85 %) Base isoliert, die säulenchromatographisch gereinigt wurde.

| | | | |
|---|---|---|---|
| Analyse: ber. | C 72,1 | H 9,7 | N 6,7 |
| gef. | C 72,0 | H 9,8 | N 6,8 |

In analoger Weise erhielt man:

13) (S)-5-[N-Methyl-N-(2-cyclohexyl)ethyl]amino-2-(3,4,5-trimethoxyphenyl)-2-isopropyl-valeronitril-hydrochlorid
Fp. 175-176 ° C,

$$[\alpha]_{589}^{20} = -9,5^0$$

(c = 10 mg/ml EtOH, d = 10 cm)

14) (R)-5-[N-Methyl-N-(2-cyclohexyl)ethyl]amino-2-(3,4,5-trimethoxyphenyl)-2-isopropyl-valeronitril-hydrochlorid
Fp. 175-176 ° C,

$$[\alpha]_{589}^{20} = +9,1^0$$

(c = 10 mg/ml EtOH, d = 10 cm)

15) 5-[N-Methyl-N-(n-octen-3-yl)]amino-2-(3,4,5-trimethoxyphenyl)-2-isopropyl-valeronitril-hydrochlorid
Fp. 176 ° C

16) 5-[N-Methyl-N-(2-cyclohexyl)ethyl]amino-2-(2-nitro-3,4-dimethoxyphenyl)-2-isopropyl-valeronitril

| | | | |
|---|---|---|---|
| Analyse: ber. | C 67,4 | H 8,8 | N 9,4 |
| gef. | C 67,5 | H 8,7 | N 9,4 |

17) 5-[N-Methyl-N-(2-cyclohexyl)ethyl]amino-2-(4-nitro-3,5-dimethoxyphenyl)-2-isopropyl-valeronitril

| | | | |
|---|---|---|---|
| Analyse: ber. | C 67,4 | H 8,8 | N 9,4 |
| gef. | C 67,2 | H 8,8 | N 9,3 |

(18) 5-{N-Methyl-N-[2-(cyclohexen-1-yl)ethyl]}amino-2-phenyl-2-isopropyl-valeronitril

| | | | |
|---|---|---|---|
| Analyse: ber. | C 81,6 | H 10,1 | N 8,3 |
| gef. | C 81,8 | H 9,8 | N 8,4 |

19) 5-[N-Methyl-N-(2-cyclohexyl)ethyl]amino-2-(3,4-dimethoxyphenyl)-2-isopropyl-valeronitril-hydrochlorid, Fp. 131-132 ° C

20) 5-{N-Methyl-N-[2-(cyclohexen-1-yl)ethyl]}amino-2-(3,4,5-trimethoxyphenyl)-2-isopropyl-valeronitril-hydrochlorid, Fp. 165-167 ° C

21) 5-{N-Methyl-N-[2-(cyclohexen-1-yl)ethyl]}amino-2-(3,4-dimethoxyphenyl)-2-isopropyl-valeronitril

| Analyse: ber. | C 75,3 | H 9,6 | N 7,0 |
|---|---|---|---|
| gef. | C 75,2 | H 8,9 | N 7,0 |

22) 5-[N-Methyl-N-(n-nonyl)]amino-2-(3,4,5-trimethoxyphenyl)-2-isopropyl-valeronitril-hydrochlorid, Fp. 142-143 °C

23) 5-[N-Methyl-N-(n-hexyl)]amino-2-(3,4,5-trimethoxyphenyl)-2-isopropyl-valeronitril-hydrochlorid, Fp. 150-152 °C

24) (S)-5-[N-Methyl-N-(n-hexyl)]amino-2-(3,4,5-trimethoxyphenyl)-2-isopropyl-valeronitril-hydrochlorid, Fp. 143-145 °C,

$$[\alpha]^{20}_{589} = -9,0^0$$

(c = 10 mg/ml, EtOH, d = 10 cm)

25) (R)-5-[N-Methyl-N-(n-hexyl)]amino-2-(3,4,5-trimethoxyphenyl)-2-isopropyl-valeronitril-hydrochlorid, Fp. 143-145 °C,

$$[\alpha]^{20}_{589} = -9,0^0$$

(c = 10 mg/ml, EtOH, d = 10 cm)

26) (S)-5-[N-Methyl-N-(2-cyclohexylethyl)]amino-2-phenyl-2-isopropyl-valeronitril-hydrochlorid Fp. 180-183 °C,

$$[\alpha]^{20}_{589} = -9,4^0,$$

(c = 10 mg/ml EtOH, d = 10 cm)

27) (R)-5-[N-Methyl-N-(2-cyclohexylethyl)]amino-2-phenyl-2-isopropyl-valeronitril-hydrochlorid Fp. 180-183 °C,

$$[\alpha]^{20}_{589} = +9,4^0,$$

(c = 10 mg/ml EtOH, d = 10 cm)

28) 5-{N-[2-(Cyclohexen-1-yl)ethyl]}amino-2-isopropyl-2-phenyl-valeronitril-hydrochlorid, Fp. 180-182 °C

29) 5-[N-2-(Cyclohexyl)ethyl-N-methyl]amino-2,2-diphenyl-valeronitril-hydrochlorid, Fp. 82-84 °C

30) 5-[N-2-(Cyclohexyl)ethyl-N-methyl]amino-2-(cyclohexen-1-yl)-2-phenyl-valeronitril-hydrochlorid, Fp. 150-152 °C

Analog können hergestellt werden:
4-[N-Methyl-N-(n-octyl)]amino-2-(3,5-dimethoxyphenyl)-2-isopropyl-butyronitril,
6-[N-(n-Heptyl)-N-methyl]amino-2-(n-propyl)-2-(3,4,5-trimethoxyphenyl)-capronitril,
5-[N-(n-Butyl)-N-(n-hexyl)]amino-2-isopropyl-2-(3-methoxyphenyl)-valeronitril,
5-(N-Ethyl-N-(n-undecyl)amino-2-isopropyl-2-(3,4,5-trimethoxyphenyl)-valeronitril,
5-[N-(2-Cyclohexyl)ethyl-N-methyl]amino-2-isopropyl-2-(3-trifluormethylphenyl)-valeronitril,
5-[N-(n-Dodecyl)-N-methyl]amino-2-(4-chlorphenyl)-2-allyl-valeronitril,
5-[N-(2-Cyclohexyl)ethyl-N-methyl]amino-2-(3-fluorphenyl)-2-isopropyl-valeronitril,
5-[N-Methyl-N-(n-octyl)]amino-2-isopropyl-2-(m-tolyl)-valeronitril,
5-[N-Methyl-N-(n-nonyl)]amino-2-(4-ethylphenyl)-2-isopropyl-valeronitril,
5-[N-(2-Cyclohexyl)ethyl-N-methyl]amino-2-(4-tert.butylphenyl)-2-isopropyl-valeronitril,
5-[N-Methyl-N-(n-octyl)]amino-2-isopropyl-2-(1,2,3,4-tetrahydronaphthyl-2)-valeronitril,
5-[N-(2-Cyclohexyl)ethyl-N-methyl]amino-2-isopropyl-2-(3,4-methylendioxyphenyl)-valeronitril,
5-[N-Methyl-N-(n-hexyl)]amino-2-(1,3-benzodioxan-6-yl)-2-isopropyl-valeronitril,
5-[N-Ethyl-N-(n-octyl)]amino-2-(1,4-benzodioxan-6-yl)-2-isopropyl-valeronitril

Beispiel A

Auf einer Tablettenpresse werden in üblicher Weise Tabletten folgender Zusammensetzung gepreßt:
40 mg Substanz des Beispiels 11
120 mg Maisstärke
13,5 mg Gelatine
45 mg Milchzucker
2,25 mg Aerosil® (chemisch reine Kieselsäure in submikroskopisch feiner Verteilung)
6,75 mg Kartoffelstärke (als 6 %iger Kleister).

Beispiel B

In üblicher Weise werden Dragees folgender Zusammensetzung hergestellt:
20 mg Substanz des Beispiels 11
60 mg Kernmasse
60 mg Verzuckerungsmasse
Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm.Ind. 1962, 586). Die Verzuckerungsmasse besteht aus 5 Teilen Rohzucker, 2 Teilen Maisstärke, 2 Teilen Calciumcarbonat und 1 Teil Talk. Die so hergestellten Dragees werden anschließend mit einem magensaftresistenten Überzug versehen.

Beispiel C

10 g Substanz des Beispiels 11 werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

**Patentansprüche**
**Patentansprüche für folgende Vertragsstaaten: AT, BE, CH, FR, GB, IT, LI, NL, SE**

**1.** Basisch substituierte Phenylacetonitrile der Formel I

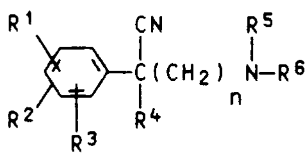

I,

worin

| | |
|---|---|
| $R^1$, $R^2$, $R^3$ | gleich oder verschieden sind und Wasserstoffatome, Halogenatome, Trifluormethylgruppen, $C_1$-$C_4$-Alkylgruppen, Nitrogruppen oder $C_1$-$C_4$-Alkoxygruppen bedeuten, wobei auch jeweils zwei Reste in Nachbarstellung zusammen Methylendioxy-, Ethylendioxy-, 1,3-Dioxatetramethylen-, Propylen- oder Butylengruppen bilden können, |
| $R^4$ | eine gesättigte oder ungesättigte Alkyl- oder Cycloalkylgruppe mit bis zu 8 C-Atomen oder eine Phenylgruppe ist, |
| $R^5$ | ein Wasserstoffatom oder einen $C_1$-$C_4$-Alkylrest darstellt, |
| $R^6$ | einen gesättigten oder ungesättigten Kohlenwasserstoffrest mit 6 bis 15 Kohlenstoffatomen, der gegebenenfalls einen nicht-aromatischen Ring enthalten kann, darstellt und |
| n | die Zahl 2, 3 oder 4 bedeutet, |

sowie deren Antipoden und Salze mit physiologisch verträglichen Säuren.

**2.** 5-[N-Methyl-N-(n-octyl)]amino-2-(3,4,5-trimethoxyphenyl)-2-isopropyl-valeronitril und seine Enantiomeren

**3.** 5-[N-Methyl-N-(n-hexyl)]amino-2-(3,4,5-trimethoxyphenyl)-2-isopropyl-valeronitril und seine Enantiomeren

**4.** 5-[N-Methyl-N-(2-cyclohexyl)ethyl]amino-2-(3,4,5-trimethoxyphenyl)-2-isopropyl-valeronitril und seine Enantiomeren

**5.** 5-[N-Methyl-N-(2-cyclohexyl)ethyl]amino-2-phenyl-2-isopropyl-valeronitril und seine Enantiomeren

**6.** 5-{N-Methyl-N-[2-(cyclohexen-1-yl)ethyl]}amino-2-(3,4,5-trimethoxyphenyl)-2-isopropyl-valeronitril und seine Enantiomeren

**7.** 5-{N-Methyl-N-[2-(cyclohexen-1-yl)ethyl]}amino-2-phenyl-2-isopropyl-valeronitril und seine Enantiomeren

**8.** Verfahren zur Herstellung der basisch substituierten Phenylacetonitrile der Formel I gemäß Anspruch 1, dadurch gekennzeichnet, daß man
a) Phenylacetonitrile der Formel II

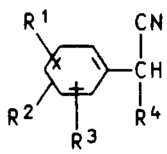

II,

worin $R^1$ bis $R^4$ die angegebene Bedeutung haben, mit Aminen der Formel III

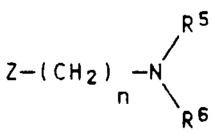

III,

worin $R^5$, $R^6$ und n die angegebene Bedeutung haben und Z eine Austrittsgruppe darstellt, umsetzt oder
b) basisch substituierte Phenylacetonitrile der Formel IV

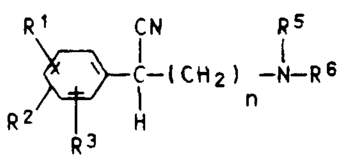

IV,

worin $R^1$ bis $R^3$, $R^5$, $R^6$ und n die angegebene Bedeutung haben mit Verbindungen der Formel V

$R^4$-Z V,

worin $R^4$ und Z die vorstehende Bedeutung haben, zur Reaktion bringt oder c) Phenylacetonitrile der Formel VI

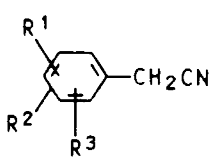

VI,

worin $R^1$ bis $R^3$ die angegebene Bedeutung haben, mit Aminen der Formel III und Verbindungen der Formel V umsetzt oder

d) Phenylacetonitrile der Formel VII

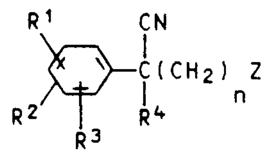

VII,

worin $R^1$ bis $R^4$, n und Z die oben angegebene Bedeutung haben, mit einem Alkylamin der Formel VIII

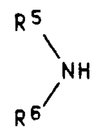

VIII,

worin $R^5$ und $R^6$ die obige Bedeutung haben, zur Reaktion bringt oder
e) Phenylacetonitrile der Formel IX

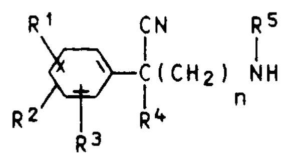

IX,

bzw. der Formel X

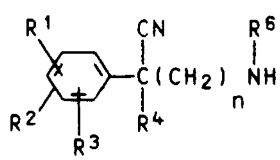

X,

worin $R^1$ bis $R^6$ und n die beschriebene Bedeutung haben, mit Verbindungen der Formel XI bzw. XII

XI $R^6$-Z bzw. XII $R^5$-Z

worin $R^5$, $R^6$ und Z die oben angegebene Bedeutung (ausgenommen $R^5$ = H) haben, zur Reaktion bringt oder
f) - falls die Reste $R^5$ und $R^6$ eine mit dem Stickstoffatom verbundene $CH_2$-Gruppe enthalten - Phenylacetonitrile der Formel IX bzw. X mit Aldehyden der Formel XIII bzw. XIV

XIII $R^7$-CHO bzw. XIV $R^8$-CHO

wobei $R^7$ bzw. $R^8$ so definiert sind, daß $R^7$-$CH_2$ gleich $R^5$ und $R^8$-$CH_2$ gleich $R^6$ bedeuten, unter reduktiven Bedingungen umsetzt oder

g) Aldehyde der Formel XV

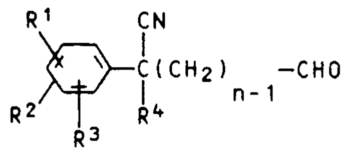

XV,

wobei $R^1$ bis $R^4$ und n dasselbe wie oben bedeuten mit Alkylaminen der Formel VIII unter reduktiven Bedingungen umsetzt oder

h) Dinitrile der Formel XVI

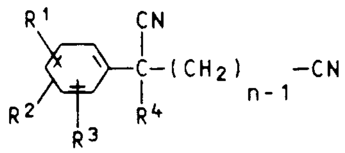

XVI,

worin $R^1$ bis $R^4$ und n dasselbe wie oben bedeuten in Gegenwart eines Alkylamins der Formel VIII reduziert oder

i) Nitrile der Formel XVII bzw. XVIII

$R^7$-CN XVII bzw. $R^8$-CN XVIII

wobei $R^7$ bzw. $R^8$ wie vorstehend definiert sind und $R^7$ nicht Wasserstoff bedeuten kann, mit Phenylacetonitrilen der Formel IX bzw. X hydriert oder

k) - falls $R^6$ eine mit dem Stickstoffatom verbundene $CH_2$-Gruppe enthält -Phenylacetonitrile der Formel XIX

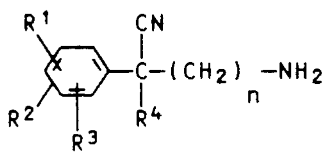

XIX,

worin $R^1$ bis $R^4$ und n dasselbe wie oben bedeuten, in Gegenwart von Nitrilen der Formel XVIII hydriert

und in den so erhaltenen Verbindungen, falls $R^5$ ein Wasserstoffatom darstellt, gewünschtenfalls eine $C_1$-$C_4$-Alkylgruppe einführt und anschließend die Verbindungen gegebenenfalls in ihre Salze mit physiologisch verträglichen Säuren überführt.

**9.** Verbindungen der Formel I gemäß Anspruch 1 zur Verwendung bei der Bekämpfung von Krankheiten.

**10.** Verwendung der Verbindungen der Formel I gemäß Anspruch 1 bei der Bekämpfung von Herzkreislauferkrankungen und asthmatischen Erkrankungen.

**11.** Verwendung der Verbindungen der Formel I gemäß Anspruch 1 bei der Bekämpfung von Magen- und Duodenalulcera.

**Patentanspruch für folgenden Vertragsstaat: ES**

1. Verfahren zur Herstellung der basisch substituierten Phenylacetonitrile der Formel I

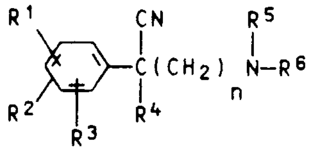

I,

worin

$R^1$, $R^2$, $R^3$ gleich oder verschieden sind und Wasserstoffatome, Halogenatome, Trifluormethylgruppen, $C_1$-$C_4$-Alkylgruppen, Nitrogruppen oder $C_1$-$C_4$-Alkoxygruppen bedeuten, wobei auch jeweils zwei Reste in Nachbarstellung zusammen Methylendioxy-, Ethylendioxy-, 1,3-Dioxatetramethylen-,Propylen- oder Butylengruppen bilden können,

$R^4$ eine gesättigte oder ungesättigte Alkyl- oder Cycloalkylgruppe mit bis zu 8 C-Atomen oder eine Phenylgruppe ist,

$R^5$ ein Wasserstoffatom oder einen $C_1$-$C_4$-Alkylrest darstellt,

$R^6$ einen gesättigten oder ungesättigten Kohlenwasserstoffrest mit 6 bis 15 Kohlenstoffatomen, der gegebenenfalls einen nicht-aromatischen Ring enthalten kann, darstellt und

n die Zahl 2, 3 oder 4 bedeutet,

sowie deren Antipoden und Salze mit physiologisch verträglichen Säuren, dadurch gekennzeichnet, daß man

a) Phenylacetonitrile der Formel II

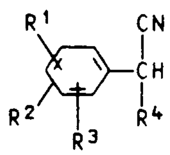

II,

worin $R^1$ bis $R^4$ die angegebene Bedeutung haben, mit Aminen der Formel III

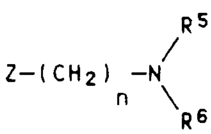

III,

worin $R^5$, $R^6$ und n die angegebene Bedeutung haben und Z eine Austrittsgruppe darstellt, umsetzt oder

b) basisch substituierte Phenylacetonitrile der Formel IV

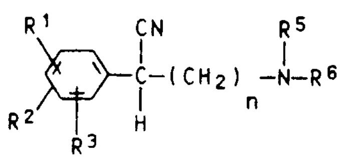

IV,

worin $R^1$ bis $R^3$, $R^5$, $R^6$ und n die angegebene Bedeutung haben mit Verbindungen der Formel V

$R^4$-Z V,

worin $R^4$ und Z die vorstehende Bedeutung haben, zur Reaktion bringt oder

c) Phenylacetonitrile der Formel VI

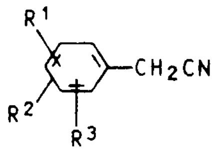

VI,

worin $R^1$ bis $R^3$ die angegebene Bedeutung haben, mit Aminen der Formel III und Verbindungen der Formel V umsetzt oder

d) Phenylacetonitrile der Formel VII

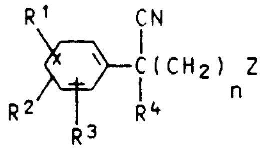

VII,

worin $R^1$ bis $R^4$, n und Z die oben angegebene Bedeutung haben, mit einem Alkylamin der Formel VIII

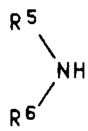

VIII,

worin $R^5$ und $R^6$ die obige Bedeutung haben, zur Reaktion bringt oder

e) Phenylacetonitrile der Formel IX

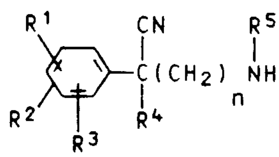

IX,

bzw. der Formel X

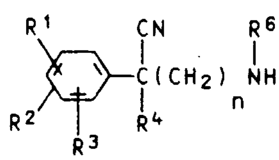

X,

worin $R^1$ bis $R^6$ und n die beschriebene Bedeutung haben, mit Verbindungen der Formel XI bzw. XII

XI $R^6$-Z bzw. XII $R^5$-Z

worin $R^5$, $R^6$ und Z die oben angegebene Bedeutung (ausgenommen $R^5$ = H) haben, zur Reaktion bringt oder

f) - falls die Reste $R^5$ und $R^6$ eine mit dem Stickstoffatom verbundene $CH_2$-Gruppe enthalten - Phenylacetonitrile der Formel IX bzw. X mit Aldehyden der Formel XIII bzw. XIV

XIII $R^7$-CHO bzw. XIV $R^8$-CHO

wobei $R^7$ bzw. $R^8$ so definiert sind, daß $R^7$-$CH_2$ gleich $R^5$ und $R^8$-$CH_2$ gleich $R^6$ bedeuten, unter reduktiven Bedingungen umsetzt oder
g) Aldehyde der Formel XV

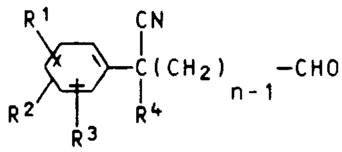
XV,

wobei $R^1$ bis $R^4$ und n dasselbe wie oben bedeuten mit Alkylaminen der Formel VIII unter reduktiven Bedingungen umsetzt oder
h) Dinitrile der Formel XVI

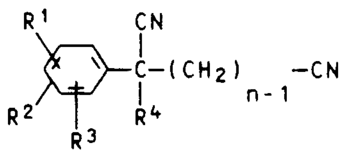
XVI,

worin $R^1$ bis $R^4$ und n dasselbe wie oben bedeuten in Gegenwart eines Alkylamins der Formel VIII reduziert oder i) Nitrile der Formel XVII bzw. XVIII

$R^7$-CN XVII bzw. $R^8$-CN XVIII

wobei $R^7$ bzw. $R^8$ wie vorstehend definiert sind und $R^7$ nicht Wasserstoff bedeuten kann, mit Phenylacetonitrilen der Formel IX bzw. X hydriert oder
k) - falls $R^6$ eine mit dem Stickstoffatom verbundene $CH_2$-Gruppe enthält - Phenylacetonitrile der Formel XIX

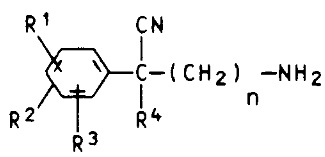
XIX,

worin $R^1$ bis $R^4$ und n dasselbe wie oben bedeuten, in Gegenwart von Nitrilen der Formel XVIII hydriert
und in den so erhaltenen Verbindungen, falls $R^5$ ein Wasserstoffatom darstellt, gewünschtenfalls eine $C_1$-$C_4$-Alkylgruppe einführt und anschließend die Verbindungen gegebenenfalls in ihre Salze mit physiologisch verträglichen Säuren überführt.

**Claims**
**Claims for the following Contracting States: AT, BE, CH, FR, GB, IT, LI, NL, SE**

**1.** A basically substituted phenylacetonitrile of the formula I

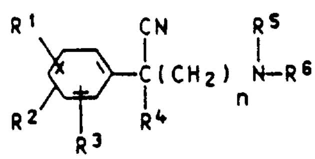
I

where $R^1$, $R^2$ and $R^3$ are identical or different and are each hydrogen, halogen, trifluoromethyl, $C_1$-$C_4$-alkyl, nitro or $C_1$-$C_4$-alkoxy, and two radicals in adjacent positions may furthermore together form a methylenedioxy, ethylenedioxy, 1,3-dioxatetramethylene, propylene or butylene group, $R^4$ is a saturated or unsaturated alkyl or cycloalkyl group of not more than 8 carbon atoms or phenyl, $R^5$ is hydrogen or $C_1$-$C_4$-alkyl, $R^6$ is a saturated or unsaturated hydrocarbon radical of 6 to 15 carbon atoms which may contain a non-aromatic ring, and n is 2, 3 or 4, and its antipodes and salts with physiologically tolerated acids.

**2.** 5-[N-methyl-N-(n-octyl)]-amino-2-(3,4,5-trimethoxyphenyl)-2-isopropylvaleronitrile and its enantiomers.

**3.** 5-[N-methyl-N-(n-hexyl)]-amino-2-(3,4,5-trimethoxyphenyl)-2-isopropylvaleronitrile and its enantiomers.

**4.** 5-[N-methyl-N-(2-cyclohexyl)ethyl]-amino-2-(3,4,5-trimethoxyphenyl)-2-isopropylvaleronitrile and its enantiomers.

**5.** 5-[N-methyl-N-(2-cyclohexyl)ethyl]-amino-2-phenyl-2-isopropylvaleronitrile and its enantiomers.

**6.** 5-{N-methyl-N-[2-(cyclohexen-1-yl)ethyl]}-amino-2-(3,4,5-trimethoxyphenyl)-2-isopropylvaleronitrile and its enantiomers.

**7.** 5-{N-methyl-N-[2-(cyclohexen-1-yl)ethyl]}-amino-2-phenyl-2-isopropylvaleronitrile and its enantiomers.

**8.** A process for the preparation of a basically substituted phenylacetonitrile of the formula I as claimed in claim 1, wherein

a) a phenylacetonitrile of the formula II

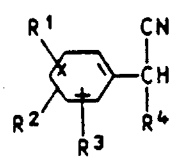

II

where $R^1$ to $R^4$ have the stated meanings, is reacted with an amine of the formula III

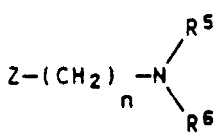

III

where $R^5$, $R^6$ and n have the stated meanings and Z is a leaving group, or

b) a basically substituted phenylacetonitrile of the formula IV

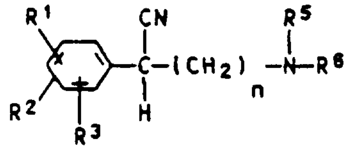

IV

where $R^1$ to $R^3$, $R^5$, $R^6$ and n have the stated meanings, is reacted with a compound of the formula V

$R^4$-Z V

where $R^4$ and Z have the above meanings, or

c) a phenylacetonitrile of the formula VI

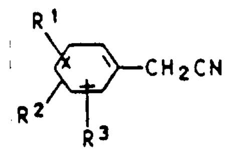

VI

where $R^1$ to $R^3$ have the stated meanings, is reacted with an amine of the formula III and a compound of the formula V, or

d) a phenylacetonitrile of the formula VII

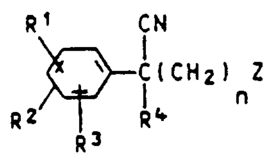

VII

where $R^1$ to $R^4$, n and Z have the abovementioned meanings, is reacted with an alkylamine of the formula VIII

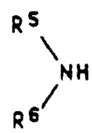

VIII

where $R^5$ and $R^6$ have the above meanings, or

e) a phenylacetonitrile of the formula IX

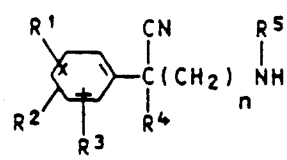

IX

or of the formula X

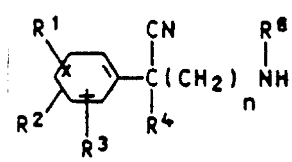

X

where $R^1$ to $R^6$ and n have the meanings described, is reacted with a compound of the formula XI or XII, respectively,

XI $R^6$-Z or XII $R^5$-Z

where $R^5$, $R^6$ and Z have the abovementioned meanings (except that $R^5$ must not be H), or

f) where $R^5$ and $R^6$ contain a $CH_2$ group bonded to the nitrogen atom, a phenylacetonitrile of the formula IX or X is reacted with an aldehyde of the formula XIII or XIV, respectively,

XIII $R^7$-CHO or XIV $R^8$-CHO

where $R^7$ and $R^8$ are defined so that $R^7$-$CH_2$ is the same as $R^5$ and $R^8$-$CH_2$ is the same as $R^6$, under reductive conditions, or

g) an aldehyde of the formula XV

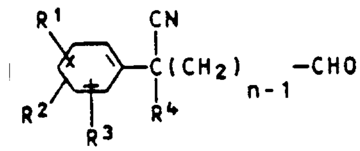

XV

where $R^1$ to $R^4$ and n have the same meanings as above, is reacted with an alkylamine of the formula VIII under reductive conditions, or

h) a dinitrile of the formula XVI

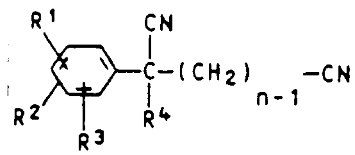

XVI

where $R^1$ to $R^4$ and n have the same meanings as above, is reduced in the presence of an alkylamine of the formula VIII, or

i) a nitrile of the formula XVII or XVIII, respectively,

$R^7$-CN XVII or $R^8$-CN XVIII

where $R^7$ and $R^8$ are as defined above and $R^7$ cannot be hydrogen, is hydrogenated with a phenylacetonitrile of the formula IX or X, or

k) where $R^6$ contains a $CH_2$ group bonded to the nitrogen atom, a phenylacetonitrile of the formula XIX

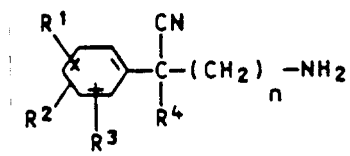

XIX

where $R^1$ to $R^4$ and n have the same meanings as above, is hydrogenated in the presence of a nitrile of the formula XVIII,

and, if desired, a $C_1$-$C_4$-alkyl group is introduced into the resulting compound if $R^5$ is hydrogen, and, if required, the compound is then converted to its salts with physiologically tolerated acids.

**9.** A compound of the formula I as claimed in claim 1 for use in the treatment of disorders.

**10.** Use of a compound of the formula I as claimed in claim 1 in the treatment of cardiovascular diseases and asthmatic disorders.

**11.** Use of a compound of the formula I as claimed in claim 1 in the treatment of peptic ulcers.

**Claim for the following Contracting State: ES**

**1.** A process for the preparation of a basically substituted phenylacetonitrile of the formula I

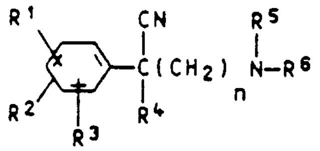

I

where $R^1$, $R^2$ and $R^3$ are identical or different and are each hydrogen, halogen, trifluoromethyl, $C_1$-$C_4$-alkyl, nitro or $C_1$-$C_4$-alkoxy, and two radicals in adjacent positions may furthermore together form a methylenedioxy, ethylenedioxy, 1,3-dioxatetramethylene, propylene or butylene group, $R^4$ is a saturated or unsaturated alkyl or cycloalkyl group of not more than 8 carbon atoms or phenyl, $R^5$ is hydrogen or $C_1$-$C_4$-alkyl, $R^6$ is a saturated or unsaturated hydrocarbon radical of 6 to 15 carbon atoms which may contain a non-aromatic ring, and n is 2, 3 or 4, and its antipodes and salts with physiologically tolerated acids, wherein

a) a phenylacetonitrile of the formula II

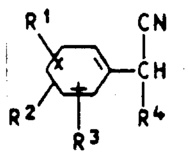

II

where $R^1$ to $R^4$ have the stated meanings, is reacted with an amine of the formula III

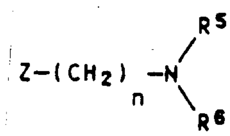

III

where $R^5$, $R^6$ and n have the stated meanings and Z is a leaving group, or

b) a basically substituted phenylacetonitrile of the formula IV

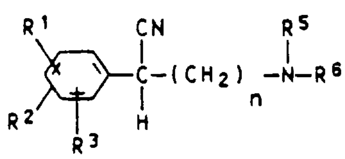

IV

where $R^1$ to $R^3$, $R^5$, $R^6$ and n have the stated meanings, is reacted with a compound of the formula V

$R^4$-Z V

where $R^4$ and Z have the above meanings, or

c) a phenylacetonitrile of the formula VI

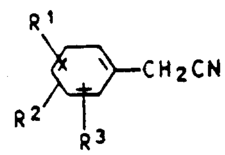

VI

where $R^1$ to $R^3$ have the stated meanings, is reacted with an amine of the formula III and a compound of the formula V, or

d) a phenylacetonitrile of the formula VII

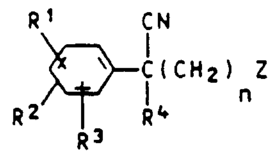

VII

where $R^1$ to $R^4$, n and Z have the abovementioned meanings, is reacted with an alkylamine of the formula VIII

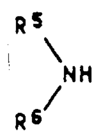

VIII

where $R^5$ and $R^6$ have the above meanings, or

e) a phenylacetonitrile of the formula IX

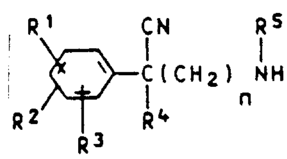

IX

or of the formula X

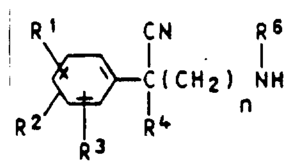

X

where $R^1$ to $R^6$ and n have the meanings described, is reacted with a compound of the formula XI or XII, respectively,

XI $R^6$-Z or XII $R^5$-Z

where $R^5$, $R^6$ and Z have the abovementioned meanings (except that $R^5$ must not be H), or

f) where $R^5$ and $R^6$ contain a $CH_2$ group bonded to the nitrogen atom, a phenylacetonitrile of the formula IX or X is reacted with an aldehyde of the formula XIII or XIV, respectively,

XIII $R^7$-CHO or XIV $R^8$-CHO

where $R^7$ and $R^8$ are defined so that $R^7$-$CH_2$ is the same as $R^5$ and $R^8$-$CH_2$ is the same as $R^6$, under reductive conditions, or
g) an aldehyde of the formula XV

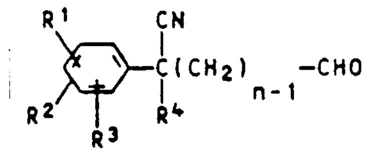

XV

where $R^1$ to $R^4$ and n have the same meanings as above, is reacted with an alkylamine of the formula VIII under reductive conditions, or
h) a dinitrile of the formula XVI

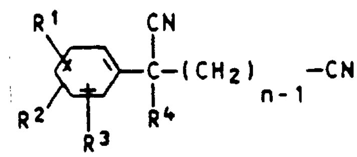

XVI

where $R^1$ to $R^4$ and n have the same meanings as above, is reduced in the presence of an alkylamine of the formula VIII, or
i) a nitrile of the formula XVII or XVIII, respectively,

$R^7$-CN XVII or $R^8$-CN XVIII

where $R^7$ and $R^8$ are as defined above and $R^7$ cannot be hydrogen, is hydrogenated with a phenylacetonitrile of the formula IX or X, or
k) where $R^6$ contains a $CH_2$ group bonded to the nitrogen atom, a phenylacetonitrile of the formula XIX

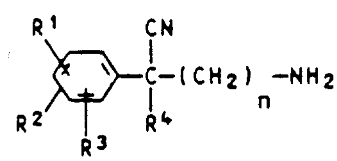

XIX

where $R^1$ to $R^4$ and n have the same meanings as above, is hydrogenated in the presence of a nitrile of the formula XVIII,
and, if desired, a $C_1$-$C_4$-alkyl group is introduced into the resulting compound if $R^5$ is hydrogen, and, if required, the compound is then converted to its salts with physiologically tolerated acids.

**Revendications**
**Revendications pour les Etats contractants suivants: AT, BE, CH, FR, GB, IT, LI, NL, SE**

**1.** Phénylacétonitriles substitués basiquement, de formule I

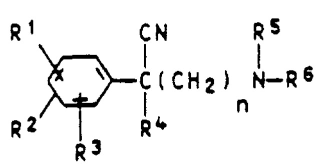

I,

dans laquelle
$R^1$, $R^2$, $R^3$ sont identiques ou différents et représentent des atomes d'hydrogène, des atomes d'halogène, des groupes trifluorométhyle, des groupes alkyle en C1-C4, des groupes nitro ou des groupes alcoxy en C1-C4, deux restes contigus pouvant aussi former, ensemble, des groupes méthylènedioxy, éthylènedioxy, 1,3-dioxatétraméthylène, propylène ou butylène,
$R^4$ est un groupe alkyle ou cycloaklyle, saturé ou insaturé, ayant jusqu'à 8 atomes C, ou un groupe phényle,
$R^5$ est un atome d'hydrogène ou un reste alkyle en C1-C4,
$R^6$ est un reste d'hydrocarbure, saturé ou insaturé, de 6 à 15 atomes de carbone, qui peut éventuellement contenir un cycle non aromtique et
n représente un des nombres 2, 3 ou 4,
ainsi que leurs antipodes et sels avec des acides physiologiquement acceptables.

**2.** 5-[N-méthyl-N-(n-octyl)]amino-2-(3,4,5-triméthoxyphényl)-2-isopropyl-valéronitrile et ses énantiomères.

**3.** 5-[N-méthyl-N-(n-hexyl)]amino-2-(3,4,5-triméthoxyphényl)-2-isopropyl-valéronitrile et ses éantiomères.

**4.** 5-[N-méthyl-N-(2-cyclohexyl)éthyl]amino-(3,4,5-triméthoxyphényl)-2-isopropyl-valéronitrile et ses énatiomères.

**5.** 5-[N-méthyl-N-(2-cyclohexyl)éthyl]amino-2-phényl-2-isopropyl-valéronitrile et ses énantiomères.

**6.** 5- {N-méthyl-N-[2-(cyclohexén-1-yl)éthyl]} amino-2-(3,4,5-triméthoxyphényl)-2-isopropyl-valéronitrile et ses énantiomères.

**7.** 5- {N-méthyl-N-[2-(cyclohexén-1-yl)éthyl]} amino-2-phényl-2-isopropyl-valéronitrile et ses énantiomères.

**8.** Procédé de préparation des phénylacétonitriles substitués basiquement, de formule I selon la revendication 1, caractérisé par le fait que l'on fait réagir
a) des phénylacétonitriles de formule II

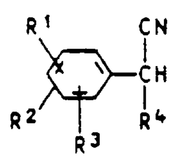

II,

dans laquelle $R^1$ à $R^4$ ont les significations données plus haut, avec des amines de formule III

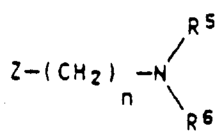

III,

dans laquelle $R^5$, $R^6$ et n ont les significations données plus haut et Z représente un groupe éliminable, ou
b) en mettant à réagir des phénylacétonitriles substitués basiquement, de formule IV

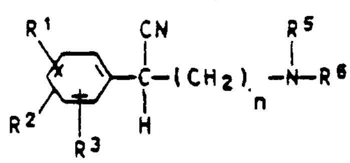

IV,

dans laquelle $R^1$ à $R^3$, $R^5$, $R^6$ et n ont les signfications données plus haut, avec des composés de formule V

$R^4$ - Z V,

dans laquelle $R^4$ et Z ont les significations données plus haut, ou
c) en faisant réagir des phénylacétonitriles de formule VI

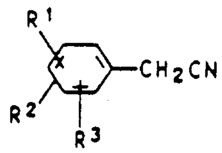

VI,

dans laquelle $R^1$ à $R^3$ ont les significations données plus haut avec des amines de formule II et des composés de formule V, ou
d) en mettant à réagir des phénylacétonitriles de formule VII

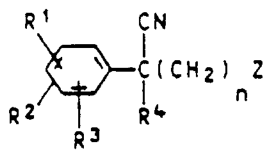

VII,

dans laquelle $R^1$ à $R^4$, n et Z ont les significations données plus haut, avec une alkylamine de formule VIII

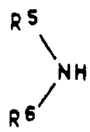

VIII,

dans laquelle $R^5$ et $R^6$ ont les significations données plus haut, ou
e) en mettant à réagir des phénylacétonitriles de formule IX

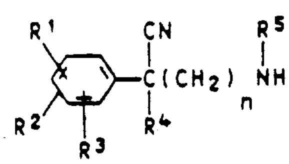

IX,

ou de formule X

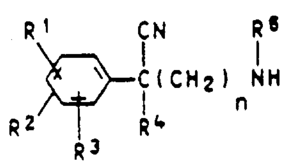

X,

où $R^1$ à $R^6$ et n ont les significations données plus haut, avec des composés de formule XI ou XII

XI $R^6$-Z ou XII $R^5$-Z,

$R^5$, $R^6$ et Z ayant les significations données plus haut (excepté $R^5$ = H), ou
f) dans le cas où les restes $R^5$ et $R^6$ contiennent un groupe $CH_2$ relié à l'atome d'azote, en mettant à réagir dans des conditions réductrices des phénylacétonitriles de formule IX ou X avec des aldéhydes de formule XIII ou XIV

XIII $R^7$-CHO ou XIV $R^8$-CHO,

où $R^7$ et $R^8$ sont définis de telle manière que $R^7$-$CH_2$ représente $R^5$ et R8-$CH_2$ représente $R^6$, ou
g) en faisant réagir, dans des conditions réductrices, des aldéhydes de formule XV

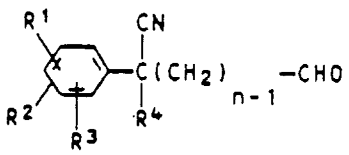

XV,

dans laquelle $R^1$ à $R^4$ et n ont les significations données plus haut, avec des alkylamines de formule VIII, ou
h) en réduisant, en présente d'une alkylamine de formule VIII, des dinitriles de formule XVI

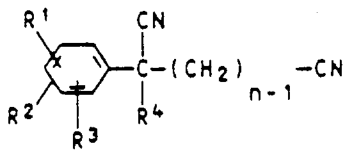

XVI,

dans laquelle $R^1$ à $R^4$ et n ont les significations données plus haut, ou
i) en hydrogénant des nitriles de formule XVII Ou XVIII

$R^7$-CN XII ou $R^8$-CN XVIII

dans lesquelles R7 et $R^8$ ont les significations données ci-dessus et $R^7$ ne peut pas reprèsenter hydrogène, avec des phénylacétonitriles de formule IX ou X, ou
k) - dans le cas où $R^6$ contient un groupe $CH_2$ relié à l'atome d'azote en hydrogénant, en présence de nitriles de formule XVIII, des phénylacétonitriles de formule XIX

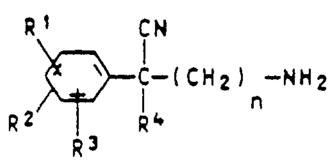

XIX,

dans laquelle $R^1$ à $R^4$ et n ont les significations données plus haut,
et, si désiré, en introduisant dans les composés ainsi obtenus, dans le cas où $R^5$ représente un atome d'hydrogène, un groupe alkyle en C1-C4, puis en transformant éventuellement les composés en leurs sels avec des acides physiologiquement acceptables.

**9.** Composés de formule I, selon la revendication 1, pour l'utilisation dans la lutte contre des maladies.

**10.** Utilisation des composés de formule I, selon la revendication 1, pour la lutte contre les troubles de la circulation et les maladies asthmatiques.

**11.** Utilisation des composés de formule I, selon la revendication 1, pour la lutte contre les ulcères de l'estomac et du duodénum.

**Revendication pour l'Etat contractant suivant: ES**

**1.** Procédé de préparation des phénylacétonitriles substitués basiquement, de formule I

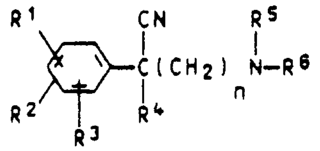

I,

dans laquelle

$R^1$, $R^2$, $R^3$ sont identiques ou différents et représentent des atomes d'hydrogène, des atomes d'halogène, des groupes trifluorométhyle, des groupes alkyle en C1-C4, des groupes nitro ou des groupes alcoxy en C1-C4, deux restes contigus pouvant aussi former, ensemble, des groupes méthylènedioxy, éthylènedioxy, 1,3-dioxatétraméthylène, propylène ou butylène,

$R^4$ est un groupe alkyle ou cycloaklyle, saturé ou insaturé, ayant jusqu'à 8 atomes C, ou un groupe phényle,

$R^5$ est un atome d'hydrogène ou un reste alkyle en C1-C4,

$R^6$ est un reste d'hydrocarbure, saturé ou insaturé, de 6 à 15 atomes de carbone, qui peut éventuellement contenir un cycle non aromtique et

n représente un des nombres 2, 3 ou 4,

ainsi que leurs antipodes et sels avec des acides physiologiquement acceptables, caractérisé par le fait que l'on fait réagir

a) des phénylacétonitriles de formule II

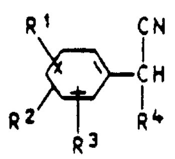

II,

dans laquelle $R^1$ à $R^4$ ont les significations données plus haut, avec des amines de formule III

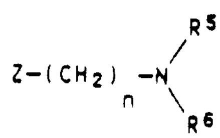

III,

dans laquelle $R^5$, $R^6$ et n ont les significations données plus haut et Z représente un groupe éliminable, ou

b) en mettant à réagir des phénylacétonitriles substitués basiquement, de formule IV

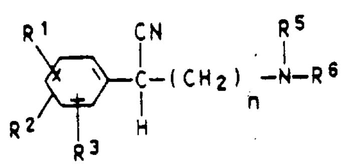

IV,

dans laquelle $R^1$ à $R^3$, $R^5$, $R^6$ et n ont les signfications données plus haut, avec des composés de formule V

$R^4$ - Z V,

dans laquelle $R^4$ et Z ont les significations données plus haut, ou

c) en faisant réagir des phénylacétonitriles de formule VI

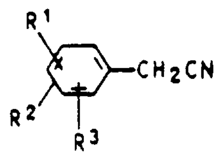

VI,

dans laquelle $R^1$ à $R^3$ ont les significations données plus haut avec des amines de formule II et des composés de formule V, ou

d) en mettant à réagir des phénylacétonitriles de formule VII

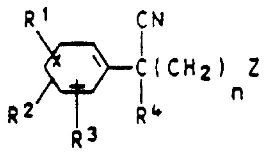

VII,

dans laquelle $R^1$ à $R^4$, n et Z ont les significations données plus haut, avec une alkylamine de formule VIII

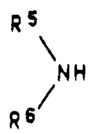

VIII,

dans laquelle $R^5$ et $R^6$ ont les significations données plus haut, ou

e) en mettant à réagir des phénylacétonitriles de formule IX

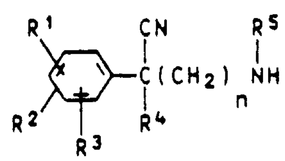

IX,

ou de formule X

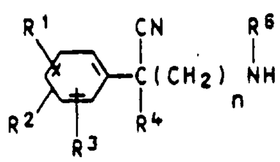

X,

où $R^1$ à $R^6$ et n ont les significations données plus haut, avec des composés de formule XI ou XII

XI $R^6$-Z ou XII $R^5$-Z,

$R^5$, $R^6$ et Z ayant les significations données plus haut (excepté $R^5$ = H), ou

f) dans le cas où les restes $R^5$ et $R^6$ contiennent un groupe $CH_2$ relié à l'atome d'azote, en mettant à réagir dans des conditions réductrices des phénylacétonitriles de formule IX ou X avec des aldéhydes de formule XIII ou XIV

XIII $R^7$-CHO ou XIV $R^8$-CHO,

où $R^7$ et $R^8$ sont définis de telle manière que $R^7$-$CH_2$ représente $R^5$ et R8-$CH_2$ représente $R^6$, ou g) en faisant réagir, dans des conditions réductrices, des aldéhydes de formule XV

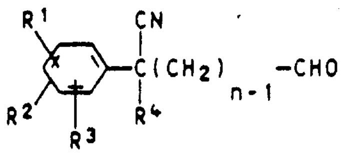

XV,

dans laquelle $R^1$ à $R^4$ et n ont les significations données plus haut, avec des alkylamines de formule VIII, ou

h) en réduisant, en présente d'une alkylamine de formule VIII, des dinitriles de formule XVI

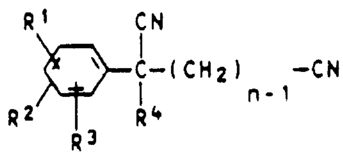

XVI,

dans laquelle $R^1$ à $R^4$ et n ont les significations données plus haut, ou

i) en hydrogénant des nitriles de formule XVII Ou XVIII

$R^7$-CN XII ou $R^8$-CN XVIII

dans lesquelles R7 et $R^8$ ont les significations données ci-dessus et $R^7$ ne peut pas représenter hydrogène, avec des phénylacétonitriles de formule IX ou X, ou

k) - dans le cas où $R^6$ contient un groupe $CH_2$ relié à l'atome d'azote en hydrogénant, en présence de nitriles de formule XVIII, des phénylacétonitriles de formule XIX

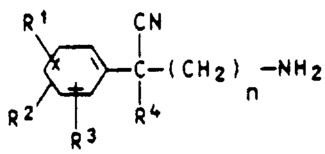

XIX.

dans laquelle $R^1$ à $R^4$ et n ont les significations données plus haut,

et, si désiré, en introduisant dans les composés ainsi obtenus, dans le cas où $R^5$ représente un atome d'hydrogène, un groupe alkyle en C1-C4, puis en transformant éventuellement les composés en leurs sels avec des acides physiologiquement acceptables.